**Europäisches Patentamt**

**European Patent Office**

**Office européen des brevets**

(19)

(11) Publication number : **0 148 145**
**B1**

(12) # EUROPEAN PATENT SPECIFICATION

(45) Date of publication of patent specification :
30.12.86

(51) Int. Cl.⁴ : **C 07 C 87/60, C 07 C 85/22**

(21) Application number : **84870162.9**

(22) Date of filing : **30.11.84**

(54) **Process for making nitrodiarylamines.**

(30) Priority : **19.12.83 US 562766**

(43) Date of publication of application :
**10.07.85 Bulletin 85/28**

(45) Publication of the grant of the patent :
**30.12.86 Bulletin 86/52**

(84) Designated contracting states :
**BE DE FR GB IT**

(56) References cited :
**DE-A- 2 850 680**
**DE-A- 2 855 764**
**DE-A- 2 909 651**
**GB-A- 855 719**

(73) Proprietor : **Monsanto Company**
**Patent Department 800 North Lindbergh Boulevard**
**St. Louis Missouri 63167 (US)**

(72) Inventor : **Wilder, Gene Ray**
**900 Damon Drive**
**Medina, Ohio 44256 (US)**
Inventor : **Merten, Helmut Ludwig**
**1660 Mayflower Lane**
**Hudson, Ohio 44236 (US)**

(74) Representative : **Lunt, John Cooper**
**Monsanto Europe S.A. Patent Department Avenue de**
**Tervuren 270-272 Letter Box No 1**
**B-1150 Brussels (BE)**

## Description

### Background of the invention

This invention relates to a method for making nitrodiarylamines, useful as intermediates for preparing dyestuffs and antidegradants. For example, 4-nitrodiphenylamine is used as an intermediate in preparing antiozonants useful in rubber compounds.

It is known to make nitrodiarylamines from nitrohaloarenes and N-acylaromatic amines or other activated forms of the amines in the presence of a so-called acid acceptor, for example, sodium carbonate. This process suffers from disadvantages, since it requires large amounts of the « acid acceptor » and large amounts of byproducts are formed.

An improved process is shown in U.S. Patent 4 187 248, in which process a nitrohaloarene, such as p-nitrochlorobenzene is reacted with a sodium, potassium, rubidium or cesium salt of the formyl derivative of an aromatic primary amine, such as potassium formanilide. The process is a batch reaction, and is exothermic, and since all of the reactants are present in the reactor from the outset, temperature and the rate of reaction are difficult to control.

### Summary of the invention

The instant invention provides an improved process for producing nitrodiarylamines. Briefly, the process involves gradually adding a sodium, potassium, rubidium or cesium salt of the formyl derivative of an aromatic primary amine to a nitrohaloarene at condensation temperature for forming nitrodiarylamine. Lithium salts are insufficiently reactive.

The process of the invention is believed to be a general one for condensation of alkali metal salts (excluding lithium salts) of formyl derivatives of aromatic primary amines, but has been examined most extensively with formanilide salts, and especially with those of formanilide itself. Salts of formanilides substituted in the benzene nucleus by one or more substituents inert under the reaction conditions, for example, one or more alkyl, alkoxy, nitro, fluoro or chloro substituents are suitable. Illustrative substituted formanilides, the sodium, potassium, rubidium or cesium salts of which may be used in the process, are 3-chloroformanilide, 4-chloroformanilide, 2-methylformanilide, 3-methylformanilide, 3-ethylformanilide, 3,4-dimethylformanilide, 3-methoxyformanilide, 4-methoxyformanilide, 4-ethylformanilide, 4-isopropylformanilide, 4-butylformanilide, 3,4-dichloroformanilide and 4-nitroformanilide.

A variety of nitrohaloarenes have been proposed for making nitrodiarylamines, any of which appear to be suitable for use in the process of the invention. Illustrative of such nitrohaloarenes are : o-nitrochlorobenzene, o-nitrobromobenzene, p-nitrochlorobenzene, p-nitrobromobenzene, m-nitrochlorobenzene, m-nitrobromobenzene, 1-chloro-2-methyl-4-nitrobenzene, 1-chloro-3-methyl-4-nitrobenzene, 1-chloro-2-nitronaphthalene, 3,4-dichloronitrobenzene, 3-methyl-4-chloronitrobenzene, 2-methyl-4-chloronitrobenzene, 2-ethyl-4-chloronitrobenzene, 2,3-dimethyl-4-chloronitrobenzene, 2,5-dimethyl-4-chloronitrobenzene, 3,5-dimethyl-4-chloronitrobenzene and p-nitrofluorobenzene.

The salt of the formyl derivative of the aromatic primary amine can be prepared in any convenient manner. U.S. Patent 4 187 248 shows several preparations of the sodium, potassium and cesium salts of formanilide. The preparation of formanilide itself is also therein described.

In a preferred mode, there is present with the nitrohaloarene in the reaction zone an amount of the formanilide which is less than equimolar with the nitrohaloarene. However, the total amount of the formanilide and its salt, together, should be at least equimolar with the nitrohaloarene, preferably in excess of an equimolar amount, and more preferably in an amount which is about a 60 percent excess. Preferred also is a diluent in the reaction zone, and especially preferred is the use of the same solvent in which the formanilide salt is contained, most preferably a hindered alcohol.

Addition of the sodium, potassium, rubidium or cesium salt of the formyl derivative of the aromatic primary amine to the reaction zone can be continuous or intermittent. Continuous addition can be at a constant rate, or at a rate which changes linearly or logarithmically. The rate of addition of the salt will determine the rate of the reaction.

The salt of the formyl derivative of the aromatic primary amine can be added neat to the nitrohaloarene, however, since the salt is usually a solid, addition is preferably accomplished with the salt in solution or suspension in a suitable carrier. Such a carrier can be solvent for the salt, and can be any such solvent which is inert in the system. Hydrocarbon solvents, such as xylene or cumene, can be used, or, preferably, more polar materials, including alcohols, dimethylformamide or dimethylsulfoxide. Especially preferred as solvents for the salt are alkyl alcohols of 1-12 carbon atoms or cycloalkyl alcohols of 4-12 carbon atoms. Among these alcohols, the most preferred are hindered alcohols, usually secondary or tertiary alcohols of 4-8 carbon atoms, such as t-butyl alcohol, t-amyl alcohol and 4-methyl-2-pentanol.

If the alcohol used is relatively volatile, it can be easily removed from the reaction zone by removing vapors and can be recovered by condensing the vapors and then separating the alcohol therefrom. The lower boiling alcohols show a tendency to promote increased nucleophilic attack on nitrohaloarenes. However, since the lower boiling alcohols can be easily removed from the reaction zone, this tendency can be minimized. The hindered alcohols are less likely to promote such an attack when they are used, even if they are allowed to remain in the reaction zone as diluents.

The process of the invention is operable at any temperature at which condensation occurs to form the nitrodiarylamine product. This temperature will be determined by the reactivity of the particular reactants chosen, and can also be determined by the speed at which the reaction can be practically performed, among other factors. Preferred reaction temperatures will range from about 100° up to about 230 °C, and more preferably between 130° and 180 °C.

Although it is not essential, it is preferred that the process of the invention be performed under agitation, to maximize the contact of the reactants with each other and to improve temperature control.

The reaction may be carried out in mild steel, stainless steel, glass or glass-lined vessels. After condensation reaches the selected end-point, the nitrodiarylamine product can be isolated in any convenient manner, such as by crystallization, distillation or a combination thereof.

## Detailed description

The process of the invention may be more clearly understood by reference to the following examples, in which all parts are by weight unless otherwise indicated.

### Example I

To a 500 ml., three-neck flask fitted with a heated dropping funnel with sidearm, a trubore paddle stirrer and two-plate packed column surmounted by a distillation head were charged 47.3 g (0.30 mole) p-nitrochlorobenzene, 19.4 g (0.16 mole) formanilide and 20 g of t-amyl alcohol. The contents of the flask were gradually heated to 145 °C, at which point dropwise addition of a solution of 57.7 g (0.33 mole) of 91.1 % potassium formanilide in 86.5 g t-amyl alcohol was begun. The solution was completely charged to the flask over a period of 137 minutes, during which time the temperature of the flask and its contents was maintained at 145°-150 °C. The flask was held at this temperature for a « hold » period of 43 additional minutes, by which time a total of 6.8 liters of carbon monoxide had been taken off. The contents of the flask were then vacuum stripped, at a pressure of 200 mm Hg (0.027 MPa), head temperature of 68 °C and a pot (flask) temperature of 105 °C. The reaction mass was then quenched with 150 ml xylene and 200 ml of 90 °C water. Another 50 ml xylene was used to wash out the flask and then added to the mother liquor in a separatory funnel. The water layer was drawn off and the xylene layer was washed a second time with 200 ml of 90° water.

The water was again separated and the xylene layer was cooled to 5 °C. The precipitated solids were filtered off and dried, yielding 44.6 g (0.208 2 mole) of 4-nitrodiphenylamine. The filtrate was analyzed to contain the following :

Aniline (0.028 7 mole), p-nitrochlorobenzene (0.026 3 mole), formanilide (0.166 4 mole), diphenylformamidine (0.004 0 mole) 4-nitrodiphenylamine (0.047 2 mole), triphenylamine (0.004 3 mole), o-nitrochlorobenzene (0.000 2 mole), p-chloroazobenzene (0.000 1 mole) and 2-nitrodiphenylamine (0.001 4 mole).

Conversion of p-nitrochlorobenzene was calculated at 91.2 %, and the yield of 4-nitrodiphenylamine at 85.1 %.

### Example II

In a manner similar to that of Example I, 4-nitrodiphenylamine was produced from formanilide, p-nitrofluorobenzene and potassium formanilide. A mixture of 30.3 g (0.25 mole) formanilide and 35.3 g (0.25 mole) p-nitrofluorobenzene was charged to the flask, and heated with agitation under a nitrogen blanket to 160° ± 5 °C. A solution of 49.3 g (0.25 mole) of potassium formanilide in 200 ml of t-amyl alcohol was added dropwise over a 70 minute period, during which the head temperature stayed at about 100°. Heating was continued for another 15 minutes, at which time the reaction was quenched with 150 ml xylene, then 100 ml $H_2O$. The reaction mass was cooled to 90° and the organic layer separated and then dried by azeotropic distillation. A yield of 74.54 % was calculated, and a conversion of 89.93 %.

### Example III

Similarly, 4-nitrodiphenylamine was prepared from p-nitrochlorobenzene, formanilide and sodium formanilide, as follows : 52.6 g (0.30 mole) sodium formanilide was suspended in 400 ml t-amyl alcohol and added dropwise to a stirred mixture of 47.3 g (0.30 mole) p-nitrochlorobenzene and 36.3 g (0.30 mole) formanilide maintained at 185° ± 5 °C. Evolved CO was measured, and the t-amyl alcohol was collected as it distilled out through a two-plate Oldershaw column connected to the distillation head. After 65 minutes all the suspension had been added and the reaction mass was kept at temperature for an additional 15 minutes. The alcohol was then returned to the pot, quenching the reaction and lowering the temperature of the reaction mass to about 120 °C. About 250 ml of alcohol was distilled off, the residue cooled, and 100 ml $H_2O$ and 100 ml xylene added thereto. The organic layer was then separated from the aqueous layer, and the former was heated to distill off about 200 ml of wet xylene and alcohol. The organic layer was cooled and filtered yielding 13.9 g (0.064 9 mole) 4-nitro-diphenylamine. The filtrate (168.0 g) contained 13.4 g p-nitrochlorobenzene and 4.87 g 4-nitrodiphenylamine. Overall yield calculated as 47.4 %, conversion, 53.3 %.

### Example IV

In a similar manner, 4-nitro-4'-methyldiphenylamine was prepared by the reaction of 4-methyl formanilide, potassium salt, with p-nitrochlorobenzene. The pot temperature was maintained at 175° ± 5 °C, with 34.4 g (0.25 mole) p-nitrochlorobenzene and 33.8 g (0.25 mole) 4-methyl formanilide stirred under nitrogen. A suspension of 56.5 g (0.25 mole) potassium 4-methyl formanilide in 250 ml of t-amyl alcohol was added dropwise over a 75 minute period. The temperature was maintained for an additional 30 minutes, and the reaction was then quenched with 150 ml xylene, then 100 ml H₂O. The layers were separated and the organic layer was dried by azeotropic distillation to a head temperature of 140°. The residue was cooled and 13.2 g (0.058 mole) solid 4-nitro-4'-methyldiphenylamine was recovered. Additional product recovered from the filtrate brought the total yield to 38.4 %, at a conversion of 41.0 %.

Example V

Similarly, 4-nitro-4'-chloro-diphenylamine was prepared from the potassium salt of 4-chloroformanilide and p-nitrochlorobenzene. Again, the reaction was run at 175° ± 5 °C, for 80 minutes, plus a fifteen-minute hold time. On recovery, 80.9 % conversion was accomplished, with a 4-nitro-4'-chloro-diphenylamine yield of 59.6 %.

Example VI

Similarly, 4-nitrodiphenylamine was prepared from rubidium formanilide and p-nitrochlorobenzene, by the dropwise addition of a t-amyl alcohol suspension of rubidium formanilide to a mixture of formanilide and p-nitrochlorobenzene, at 175° ± 5 °C. The product was recovered as before, giving a conversion of 77.7 % and a 4-nitrodiphenylamine yield of 66.1 %.

Example VII

Cesium formanilide was used in preparing 4-nitrodiphenylamine as before. A solution of cesium formanilide in t-amyl alcohol was added dropwise to a mixture of formanilide and p-nitrochloro-benzene stirred and maintained at 175° ± 5 °C. The product was recovered as before, calculating to a 4-nitrodiphenylamine yield of 70.7 %, and a conversion of 77.4 %.

Example VIII

Similarly, 4-nitrodiphenylamine was prepared by a dropwise addition of potassium formanilide in solution in t-amyl alcohol to a mixture of formanilide and p-nitrofluorobenzene. The reaction was performed at 160° ± 5 °C, with addition of the solution over a 70 minute period. On recovery, as before, a yield of 75.5 % of 4-nitrodiphenylamine and a conversion of 87.9 %.

Example IX

In a similar manner, 2-nitrodiphenylamine was prepared by the dropwise addition of a solution of potassium formanilide in t-amyl alcohol to a stirred mixture of formanilide and o-nitrochlorobenzene maintained at 175° ± 5 °C. On recovery, as before, a 2-nitrodiphenylamine yield of 74.7 % and a conversion of 85.1 % were measured.

Although the invention has been illustrated by typical examples, it is not limited thereto. Changes and modifications of the examples of the invention herein chosen for purposes of disclosure can be made which do not constitute departure from the spirit and scope of the invention.

**Claims**

1. The method of producing a nitrodiarylamine characterised by adding to a nitrohaloarene at condensation temperature for forming nitrodiarylamine a sodium, potassium, rubidium or cesium salt of the formyl derivative of an aromatic primary amine.

2. The method of Claim 1 wherein the formyl derivative of an aromatic primary amine is present with the nitrohaloarene.

3. The method of Claim 1 wherein the salt is added in solution in an appropriate solvent.

4. The method of Claim 3 wherein the solvent is an alkyl alcohol of 1-12 carbon atoms or a cycloalkyl alcohol of 4-12 carbon atoms.

5. The method of Claim 3 wherein the salt in solution is added intermittently.

6. The method of Claim 3 wherein the salt in solution is added continuously.

7. The method of producing a nitrodiarylamine comprising the steps of combining in a reaction zone a monohalonitrobenzene and less than a molar equivalent of a formanilide, maintaining the contents of the reaction zone at a temperature of from 100 °C to 230 °C under agitation and adding to the reaction zone a solution of a sodium, potassium, rubidium or cesium salt of formanilide, said salt being in solution in an alkyl alcohol of 1-12 carbon atoms or a cycloalkyl alcohol of 4-12 carbon atoms.

8. The method of Claim 7 wherein the contents of the reaction zone are maintained at a temperature of from 130 °C to 180 °C.

9. The method of Claim 7 wherein the alcohol is a hindered alkyl alcohol.

10. The method of producing 4-nitrodiphenylamine comprising the steps of

A) combining in a reaction zone paranitrochlorobenzene and less than a molar equivalent of formanilide at condensation temperature for forming 4-nitrodiphenylamine and

B) gradually charging to the reaction zone about a molar equivalent of a sodium, potassium, rubidium or cesium salt of formanilide, based on the paranitrochlorobenzene, said salt in solution in a hindered alkyl alcohol of from 4-12 carbon atoms.

11. The method of Claim 10 wherein the formanilide salt is potassium formanilide.

12. The method of Claim 10 wherein the alcohol is 4-methyl-2-pentanol.

13. The method of Claim 10 wherein the reaction zone is maintained at a temperature of from 100° to 230 °C.

14. The method of Claim 13 wherein the reaction zone is maintained at a temperature of from 130° to 180 °C.

15. The method of Claim 13 wherein the alcohol is tertiary amyl alcohol.

## Patentansprüche

1. Verfahren zur Herstellung eines Nitrodiarylamins, dadurch gekennzeichnet, daß man ein Natrium-, Kalium-, Rubidium- oder Cäsiumsalz des Formylderivats eines aromatischen primären Amins bei der Kondensationstemperatur zur Bildung von Nitrodiarylamin zu einem Nitrohalogenaren zusetzt.

2. Verfahren nach Anspruch 1, dadurch gekennzeichnet, daß das Formylderivat eines aromatischen primären Amins neben dem Nitrohalogenaren vorhanden ist.

3. Verfahren nach Anspruch 1, dadurch gekennzeichnet, daß das Salz in Lösung in einem geeigneten Lösungsmittel zugegeben wird.

4. Verfahren nach Anspruch 3, dadurch gekennzeichnet, daß das Lösungsmittel ein Alkylalkohol mit 1 bis 12 Kohlenstoffatomen oder ein Cycloalkylalkohol mit 4 bis 12 Kohlenstoffatomen ist.

5. Verfahren nach Anspruch 3, dadurch gekennzeichnet, daß das gelöste Salz intermittierend zugegeben wird.

6. Verfahren nach Anspruch 3, dadurch gekennzeichnet, daß das gelöste Salz kontinuierlich zugesetzt wird.

7. Verfahren zur Herstellung eines Nitrodiarylamins, dadurch gekennzeichnet, daß man ein Monohalogennitrobenzol und weniger als 1 Mol-Äquivalent eines Formanilids in einer Reaktionszone vereinigt, den Inhalt der Reaktionszone unter Bewegen bei einer Temperatur von 100 °C bis 230 °C hält und eine Lösung eines Natrium-, Kalium-, Rubidium- oder Cäsiumsalzes von Formanilid, welches Salz in einem Alkylalkohol mit 1 bis 12 Kohlenstoffatomen oder einem Cycloalkylalkohol mit 4 bis 12 Kohlenstoffatomen gelöst ist, in die Reaktionszone einführt.

8. Verfahren nach Anspruch 7, dadurch gekennzeichnet, daß der Inhalt der Reaktionszone bei einer Temperatur von 130 °C bis 180 °C gehalten wird.

9. Verfahren nach Anspruch 7, dadurch gekennzeichnet, daß der Alkohol ein sterisch gehinderter Alkylalkohol ist.

10. Verfahren zur Herstellung von 4-Nitrodiphenylamin, gekennzeichnet durch die folgenden Schritte

A) Vereinigen von Paranitrochlorbenzol und weniger als 1 Mol-Äquivalent Formanilid bei der Kondensationstemperatur zur Bildung von 4-Nitrodiphenylamin in einer Reaktionszone und

B) graduelle Zugabe etwa eines Moläquivalents eines Natrium-, Kalium-, Rubidium- oder Cäsiumsalzes von Formanilid, bezogen auf das Paranitrochlorbenzol, welches Salz in einem sterisch gehinderten Alkylalkohol mit 4 bis 12 Kohlenstoffatomen gelöst ist, in die Reaktionszone.

11. Verfahren nach Anspruch 10, dadurch gekennzeichnet, daß das Formanilidsalz Kaliumformanilid ist.

12. Verfahren nach Anspruch 10, dadurch gekennzeichnet, daß der Alkohol 4-Methyl-2-pentanol ist.

13. Verfahren nach Anspruch 10, dadurch gekennzeichnet, daß die Reaktionszone bei einer Temperatur von 100° bis 230 °C gehalten wird.

14. Verfahren nach Anspruch 13, dadurch gekennzeichnet, daß die Reaktionszone bei einer Temperatur von 130° bis 180 °C gehalten wird.

15. Verfahren nach Anspruch 13, dadurch gekennzeichnet, daß der Alkohol ein tertiärer Amylalkohol ist.

## Revendications

1. Procédé de préparation d'une nitrodiarylamine, caractérisé en ce qu'on ajoute à un nitrohaloarène à la température de condensation pour former la nitrodiarylamine, un sel de sodium, de potassium, de rubidium ou de césium du dérivé formylé d'une amine primaire aromatique.

2. Procédé selon la revendication 1, dans lequel le dérivé formylé d'une amine primaire aromatique est présent avec le nitrohaloarène.

3. Procédé selon la revendication 1, dans lequel le sel est ajouté en solution dans un solvant approprié.

4. Procédé selon la revendication 3, dans lequel le solvant est un alcool alkylique en $C_1$ à $C_{12}$ ou un alcool cycloalkylique en $C_4$ à $C_{12}$.

5. Procédé selon la revendication 3, dans lequel le sel en solution est ajouté par intermittences.

6. Procédé selon la revendication 3, dans lequel le sel en solution est ajouté en continu.

7. Procédé de préparation d'une nitrodiarylamine comprenant les stades consistant à combiner dans une zone réactionnelle un monohalonitrobenzène et moins d'un équivalent molaire d'un formanilide, à maintenir le contenu de la zone réactionnelle à une température de 100 à 230 °C en agitant et à ajouter à la zone réactionnelle une solution d'un sel de sodium, de potassium, de rubidium ou de césium du formanilide, ce sel étant en solution dans un alcool alkylique en $C_1$ à $C_{12}$ ou dans un alcool cycloalkylique en $C_4$ à $C_{12}$.

8. Procédé selon la revendication 7, dans lequel le contenu de la zone réactionnelle est maintenu à une température de 130 à 180 °C.

9. Procédé selon la revendication 7, dans lequel l'alcool est un alcool alkylique encombré.

10. Procédé de préparation d'une 4-nitrodiphénylamine comprenant les étapes consistant à :

A) combiner dans une zone réactionnelle du paranitrochlorobenzène et moins d'un équivalent molaire de formanilide à la température de condensation pour former la 4-nitrodiphénylamine et,

B) à introduire progressivement dans la zone réactionnelle environ un équivalent molaire d'un sel de sodium, de potassium, de rubidium ou de césium du formanilide, par rapport au paranitrochlorobenzène, ce sel étant en solution dans un alcool alkylique encombré en $C_4$ à $C_{12}$.

11. Procédé selon la revendication 10, dans lequel le sel de formanilide est le formanilide de potassium.

12. Procédé selon la revendication 10, dans lequel l'alcool est le 4-méthyl-2-pentanol.

13. Procédé selon la revendication 10, dans lequel la zone réactionnelle est maintenue à une température de 100 à 230 °C.

14. Procédé selon la revendication 13, dans lequel la zone réactionnelle est maintenue à une température de 130 à 180 °C.

15. Procédé selon la revendication 13, dans lequel l'alcool est l'alcool amylique tertiaire.